# EUROPEAN PATENT APPLICATION

(11) **EP 4 066 668 A1**
(43) Date of publication of application: **05.10.2022**
(21) Application number: 20905499.8
(22) Date of filing: 27.12.2020
(51) Int. Cl.: A41D 13/11, A62B 18/08, A45D 44/22, H04R 1/08, A42B 1/00, A42B 3/08, H04R 1/10

(54) **APPARATUS FIXED TO FACE**

(30) Priority: 27.12.2019 KR 20190177090; 12.06.2020 KR 20200071798; 24.12.2020 KR 20200183985
(71) Applicant: Koreatechnics Co., Ltd., Seoul 06103 (KR)
(72) Inventor: LEE, Dong Yol, Seoul 06793 (KR)
(74) Representative: Plasseraud IP
(86) International application number: PCT/KR2020/019166
(87) International publication number: WO 2021/133131

(57) **Abstract**

The invention of the present application relates to an apparatus fixed to the face. Particularly, the present invention relates to an apparatus which is fixed to the face by bringing, in close contact with a specific part of the face, at least one from among a mask, hat, helmet, portable sound equipment, glasses or goggles, eye patch, VR device, chin strap and cosmetic facial mask.

## Description

### [Technical Field]

This application claims the benefit of priority to Korean Patent Application No. 2019-0177090 filed on December 27, 2019, Korean Patent Application No. 2020-0071798 filed on June 12, 2020, and Korean Patent Application No. 2020-0183985 filed on December 24, 2020 with the Korean Intellectual Property Office, the disclosures of which are incorporated herein by reference in their entireties.

The present invention relates to an apparatus fixed to a face. More particularly, the present invention relates to an apparatus fixed to a face of a user as the result of being brought into tight contact with a specific region of the face, wherein the apparatus is at least one of a mask, a hat, a helmet, a portable sound device, glasses or goggles, an eye bandage, a VR device, a jaw assist device, and a cosmetic facial support.

### [Background Art]

Apparatuses worn on a face using ears as a support fixture have been greatly increased. Since COVID-19, a mask has become an everyday product. Not only traditional glasses but also a headphone or an earphone uses ears as a support fixture in various manners. Utilization of a VR device, which is a product using ears as a support fixture, has gradually increased.

Time for which apparatuses using ears as a support fixture are worn has also greatly increased. In addition to glasses, which are worn all day long, a mask is worn irrespective of place and time. Using an earphone having new technology applied thereto for several hours has become very natural.

As a result, it is expected that physical stress on ears will greatly increase. Discomfort will be increased for glasses wearers, which are more than 50% of adults. Everyday wearing of a mask due to COVID-19 and fine dust is one of the factors that increase physical burden on ears.

Therefore, there is a need to develop a new-concept apparatus or method capable of fixing various kinds of devices to a face while reducing physical stress on ears. It is necessary to develop a device that can be fixed to a second fixing region capable of assisting ears or replacing the ears or a method of fixing the device to the second fixing region, not a conventional method relying only on the ears.

In order to solve such a problem, the inventor of the present application has analyzed problems with a mask, which is a representative example of an apparatus fixed to a face. To this end, preliminary research on a leakage rate depending on the length of a string of the mask and pain felt when the mask is worn was conducted. In the present invention, preliminary research on a KF94 mask was conducted for nine participants.

First, research on appropriate length of the string of the mask capable of achieving a low leakage rate, which is a basic object of the mask, was conducted. A leakage rate in the case in which the length of a string of a mask manufactured for adults was not adjusted and a leakage rate in the case in which the length of the mask including the string was adjusted to 120% of a facial length were compared with each other. It was revealed that, in the latter case, the leakage rate was reduced to at least 25% and at most 83%, compared to the former case. That is, it can be seen that, only when the length of the string of the mask is adjusted, it is possible to reduce the leakage rate, whereby it is possible to achieve the original purpose of wearing the mask.

Here, the facial length is a facial length measured using Morpheus and is defined as the distance from the left ear to the position under the nose of a user and from the position under the nose to the right ear of the user.

When the length of the string of the mask is adjusted, the leakage rate may be reduced; however, pain in various regions due to long-term wearing was observed.

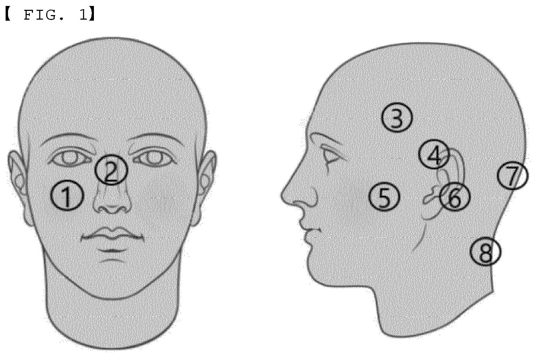

FIG. 1 is a schematic view showing positions at which pains depending on wearing of a mask were observed. Qualitative evaluation of the degree of pain in specific portions of a face shown in FIG. 1 immediately after the mask was worn and after the mask was continuously worn for eight hours was performed. It was observed that, when the mask was continuously worn for eight hours without adjusting the length of a string of the mask, the degree of pain was greatly increased only at region 6 (behind the ear). This is analyzed as pain that appears as the result of the string of the mask being continuously fixed to the ears even though the degree of pressure against the face is low.

It was revealed that, when the mask was continuously worn for eight hours in the state in which the leakage rate was reduced by adjusting the length of the string of the mask to 120% of the facial length, pain increased at various regions, including region 6. It was revealed that pain also greatly increased at region 3 and region 7, including region 4 related to the ear. Region 3 and region 7 are regions at which pain of a fascia tender point is manifested, and headache may be easily caused by this pain. The temporalis muscle and muscles of an upper cervical vertebrae extensor of an occipital region, which correspond to region 3 and region 7, respectively, are regions which cause pain of a fascia tender point of an upper trapezius, and headache and shoulder pain may be caused.

The inventor of the present application has found through preliminary research that, when the mask was worn for a long time, pain was also caused in musculoskeletal systems other than the ears. Various kinds of pain caused due to wearing of the mask also cause lack of concentration, whereby study or work may be impeded.

The present invention has been made in view of the above problems, and the present invention has been derived through a process of developing an apparatus fixed to a face that is capable of being fixed to a second fixing region excluding the ears.

Patent Document 1 relates to a mask that is easily and comfortably worn, the mask including a frame 20 including a holding portion 21 held over a bent portion of a front surface of a face and an elastic tight contact portion 22 extending from the holding portion 21 so as to come into contact with an upper end of a zygomatic bone and extending to a side surface of the face near each ear along the contour of the face, a main body 10 having formed therein a mounting hole 11, into which the frame 20 is detachably inserted, the main body being configured to wrap the vicinities of a nose and a mouth of the face, the main body being made of an elastic fiber band, and a filter 30 detachably mounted to an inner surface of the main body 10, the filter having formed therein a mounting hole 31, into which the frame 20 is detachably inserted, the filter being configured to wrap alar creases and the mouth, wherein the holding portion 21 and the elastic tight contact portion 22 are maintained in a state of being held over the face in tight contact with the face due to elastic force of the elastic tight contact portion 22 of the frame 20. An object of Patent Document 1 is to provide a mask that is stably held over a face of a user without discomfort in ears and that has improved convenience in use. The mask is configured to have a structure in which the main body and the filter are detachably attached to each other, whereby it is possible to replace the filter, and the main body and the filter may be separated from each other so as to be independently used depending on a use environment.

In Patent Document 1, the frame 20 including the elastic tight contact portion 22 extending from the holding portion 21 so as to come into contact with the upper end of the zygomatic bone and extending to the side surface of the face near each ear along the contour of the face is widely disposed over a region including the vicinity of a cheek. In this case, 1) the face may be rubbed during attachment and detachment of the mask, whereby the skin may be irritated or makeup may be removed. In addition, 2) the mask continuously irritates a weak region of the skin, whereby a mark is left on a face of a user when the user wears the mask for a long time, and the user feels discomfort. Furthermore, 3) definite criteria for a surface that is pressed and pressure applied thereto are not presented, whereby it is difficult to secure consistent fixing force.

Patent Document 2 relates to a neck warmer configured to block fine dust, wherein the neck warmer includes a neck cover portion formed in a cylindrical shape, the neck cover portion being configured to cover a range from positions under eyes of a face to a neck when worn, a sealing cover portion formed so as to cover a nose and a mouth of a user in a state of being spaced apart therefrom by a predetermined distance when worn, a pressing ring formed at an edge of an upper end of the neck cover portion, the pressing ring being configured to allow the upper end of the neck cover portion to come into tight contact with the face, and a filtering portion configured to filter air introduced into the nose and the mouth, whereby fine dust introduced into respiratory organs is blocked while the face and the neck are protected. An object of Patent Document 2 is to provide a functional neck warmer capable of efficiently blocking fine dust and protecting the neck and the face, wherein the neck warmer is brought into tight contact with a curved surface of the face in order to secure efficiency in blocking fine dust, and the neck warmer is prevented from moving under the chin during activity.

A typical problem with a system using a rubber band of Patent Document 2 is that 1) detachment and attachment of the neck warmer is very inconvenient. In addition, 2) the face may be rubbed during attachment and detachment of the neck warmer, whereby makeup may be removed, as in Patent Document 1. 3) A mark is left on the face of the user when the user wears the neck warmer for a long time, and the user feels discomfort.

Patent Document 3 relates to a wearable mask including a main body configured to cover a mouth region of a face of a user in order to protect the mouth region and a face fixing member connected to the main body, the face fixing member being configured to contact a part of the face of the user so as to be brought into tight contact therewith when the user wears the wearable mask. An object of Patent Document 3 is to provide a wearable mask configured to be brought into tight contact with a part of the face through a concave recess when worn on the face, whereby it is unnecessary to form a catching tool, such as a string added to the mask, and therefore it is possible to reduce manufacturing cost, and configured such that the main body is formed so as to be disposable, whereby the wearable mask is semi-permanently usable by replacing only the main body.

In Patent Document 3, 1) there is concern that the wearable mask contacts a wide region of the face, whereby makeup may be removed. In addition, 2) there is concern that a mark may be left on the face due to continuous wearing, and 3) definite criteria for a surface that is pressed and pressure applied thereto are not presented, whereby it is difficult to secure consistent fixing force.

Patent Document 4 relates to a facial mask for adults and children configured to be just fitted on a face, a jaw portion, and a front head through drawing corresponding to a solid based on a stretch rate of a material for the mask, the length from jaws to a tragus, and the position of a zygomatic bone, and an object of Patent Document 4 is to provide a drawing method corresponding to a solid of a sheet type facial mask configured to be fitted on the face, the jaw portion, and the front head and suitable for medical supplies replacing a bandage, an adhesive bandage, and an eye bandage or medical purposes and the mask.

In Patent Document 4, the mask is held by ears and tension induced therefrom is used, and therefore stress on the ears mentioned in the present invention is extremely high.

Patent Document 5 relates to a mask cover capable of being mounted to a face without ear strings, and an object of Patent Document 5 is to provide a mask configured to be deformed along a curved surface of the face using a material that exhibits excellent elasticity and including a mask cover having good breathability.

In Patent Document 5, 1) a user may feel uncomfortable wearing the mask since a separate anchor member is attached to the face, and 2) a mark is left on the face along a surface to which the anchor is attached or the skin may be injured due to continuous irritation. In addition, 3) definite criteria for the surface to which the anchor is attached are not presented, whereby it is difficult to secure consistent fixing force.

Patent Document 6, which relates to a body relaxation prevention and improvement member, provides a body relaxation prevention and improvement member configured such that manufacturing cost of the member is reduced, and the member can be mounted to a head and other regions, whereby tensile force is applied in a direction toward a face over a large range, and the relaxation prevention and improvement member is configured such that a branch capable of being wound around an occipital region is integrally connected to a stump that covers an area ranging from the top of the head or a front head to the tip of a chin and a lower part of the chin via the masseteric fascia.

Patent Document 6, in which tensile force that wraps the entirety of the face is used, is characterized by the tensile force itself, and therefore it is not possible to solve the problems presented in the present invention.

Korean Registered Patent Publication No. 10-1773856 ("Patent Document 1")

Korean Registered Patent Publication No. 10-1954022 ("Patent Document 2")

Korean Registered Utility Model Publication No. 0490095 ("Patent Document 3")

Japanese Patent Application Publication No. 2004-267692 ("Patent Document 4")

Japanese Patent Application Publication No. 2018-145570 ("Patent Document 5")

Japanese Registered Patent Publication No. 4799884 ("Patent Document 6")

### [ Disclosure]

### [ Technical Problem]

The present invention has been made in view of the above problems, and it is an object of the present invention to provide an apparatus fixed to a face configured such that 1) detachment and attachment of the apparatus are easy, 2) contact between the apparatus and the skin, including the face, during detachment and attachment of the apparatus is minimized, 3) skin irritation is low even though the apparatus is worn for a long time, 4) discomfort due to pain in other musculoskeletal systems, including the ears, is minimized, and 5) a pressed region is clearly specified and the region is optimized, whereby consistent fixing force is provided regardless of user.

### [Technical Solution]

In order to accomplish the above object, the present invention provides an apparatus fixed to a face using a common improvement method in which various kinds of devices fixed to the face can be fixed to the face of a human body.

The present invention provides an apparatus fixed to a face, the apparatus including a main body located at a front surface or a rear surface of a head of a user and
a first fixing member connected to the main body, the first fixing member being brought into tight contact with at least a part of a first fixing region of the user in order to fix the main body, wherein
in the face of the user, the first fixing region includes at least one of:
   1) a 1-1 fixing region as a lower facial region including a range from a position including a styloid process of the user to a premolar of the user in consideration of a movement range of a sagittal or anteroposterior axis and
      including a temporal process of the user in consideration of a movement range of a longitudinal or craniocaudal axis;
   2) a 1-2 fixing region as a lower facial region including a range from a position including a parotid gland of the user to a region left by excluding a region in which a funiculus anterior is located from two branches of a medial pterygoid of muscles of mastication of the user in consideration of the movement range of the sagittal or anteroposterior axis and
      including the temporal process of the user in consideration of the movement range of the longitudinal or craniocaudal axis;
   3) a 1-3 fixing region as a lower facial region including a range from the position including the styloid process of the user to a masseter muscle of the muscles of mastication of the user in consideration of the movement range of the sagittal or anteroposterior axis and
      including the temporal process of the user in consideration of the movement range of the longitudinal or craniocaudal axis, wherein
      a muscle belly, which is a most protruding portion of the masseter muscle, is excluded;
   4) a 1-4 fixing region as a lower facial region including a range from a front end of a tragus of the user to a region in which a rear surface of a zygomaticus major of the user is not pressed in consideration of the movement range of the sagittal or anteroposterior axis and
      including the temporal process of the user in consideration of the movement range of the longitudinal or craniocaudal axis;
   5) a 1-5 fixing region as an interior of a square provided based on the face of the user, wherein
      the square provided based on the face of the user is a square having an angle of mandible and a zygomatic process of the user as one surface, and wherein the square provided based on the face of the user has the sagittal or anteroposterior axis and the longitudinal or craniocaudal axis as a width and a length, respectively, and the distance from the angle of the mandible to the zygomatic process along the longitudinal or craniocaudal axis is the length of one side; and
   6) a 1-6 fixing region configured such that values of (x,y) based on the face of the user are (-1,-1), (1,-1), (2,-1), (3,-1), (4,-1), (-1,1), (1,1), (2,1), (3,1), (4,1), (5,1), (-1, 2), (1,2), (2,2), (3,2), (-1,3), (1,3), (2,3), (-1,4), (1,4), (-1,5), (1,5), and (2,5), wherein

(x,y) provided based on the face of the user is selected based on coordinates of each small square formed when the width and the length of the square provided based on the face of the user are divided into five equal parts, x is designated as 1, 2, 3, 4, and 5 from the rear surface to the front surface of the head of the user and y is designated as 1, 2, 3, 4, and 5 from an upper surface to a lower surface of the head of the user as a result of division into five equal parts,
x of the small square of the square provided based on the face of the user further disposed in symmetry based on the longitudinal or craniocaudal axis of the rear surface of the head of the user is designated as a negative value,
y of the small square of the square provided based on the face of the user further disposed in symmetry based on a lateral axis of the upper surface of the head of the user is designated as a negative value, and
x and y of the small square of the square provided based on the face of the user further disposed in symmetry based on apexes of the upper surface and the rear surface of the head of the user are designated as negative values.

Specifically, in the 1-5 fixing region, the values of (x,y) based on the face of the user may be (1,1), (2,1), (3,1), (4,1), (1,2), (2,2), (3,2), (1,3), (1,4), (1,5), and (2,5), preferably (2,2), (4,2), (1,3), (3,3), (4,3), (5,3), (1,4), (2,4), (3,4), (4,4), (5,4), (1,5), (2,5), (4,5), and (5,5), more preferably (2,1), (2,2), (1,3), (4,3), (5,3), (1,4), (2,4), (4,4), (5,4), (1,5), (2,5), and (5,5), yet more preferably (2,2), (1,3), (2,4), (1,5), and (2,5), most preferably (2,2) and (1,3).

In the 1-6 fixing region, the values of (x,y) based on the face of the user may be (-1,-1), (1,-1), (2,-1), (3,-1), (4,-1), (-1,1), (1,1), (2,1), (-1, 2), (1,2), (-1,3), and (1,3).

Meanwhile, the styloid process, the premolar, and the temporal process may be excluded from the first fixing region.

The apparatus may further include a second fixing member connected to the main body, the second fixing member being brought into tight contact with at least a part of a second fixing region of the user in order to fix the main body, wherein
the second fixing region may include at least one of a nasolabial fold area of the user, a levator labii superioris or a levator labii superioris alaeque nasi of the user, and an outer circumferential part of an alar crease of the user, and the second fixing region may include the outer circumferential part of the alar crease of the user.

At least two of the main body, the first fixing member, and the second fixing member may be made of an identical material.

The apparatus may further include an additional fixing member brought into tight contact with an additional fixing region of the face of the user other than the first fixing region and/or the second fixing region in order to fix the main body.

The apparatus may further include a first connection portion configured to connect the main body and the first fixing member to each other and a second connection portion configured to connect the main body and the second fixing member to each other, and the first connection portion and/or the second connection portion may have an adjustable length.

In the apparatus, the main body, the first connection portion, the second connection portion, the first fixing member, and the second fixing member may be integrally formed using an identical material.

The first connection portion and/or the second connection portion may be elastic.

Each of the first connection portion, the second connection portion, the first fixing member, and the second fixing member may be made of at least one of a resin, a metal, a semiconductor, ceramic, leather, paper, fiber comprising glass fiber and carbon fiber, glass, rubber, wood, a biomaterial, and carbon fiber.

The first fixing member brought into tight contact with the at least a part of the first fixing region may have at least one first tight contact surface, and the second fixing member brought into tight contact with the at least a part of the second fixing region may have at least one second tight contact surface.

The apparatus may further include an auxiliary fixing member fixed to an auxiliary fixing region located at at least one of a nose, jaws, a mastoid bone, a back of a neck, and a parietal bone or an occipital bone of the head in order to fix the main body.

The main body may be at least one of a mask, a hat, a helmet, a portable sound device, glasses or goggles, an eye bandage, a VR device, a jaw assist device, and a cosmetic facial support, and specifically
the mask may be any one of an air pollution mask, a disposable mask, a filter replaceable mask, an industrial mask, a military mask, a gas mask, a surgical mask, a cooking mask, an ultraviolet mask, a cold protection mask, a continuous positive airway pressure mask, an oxygen mask, a welding mask, a hyperventilation prevention mask, a training mask, a scuba diving mask, a skin care mask, an LED mask, an ultrasonic mask, a low frequency mask, and a facial mask,
the hat may be any one of a sun cap, a fedora, a baseball cap, and a cap,
the helmet may be any one of a bicycle helmet, a ski helmet, a snowboard helmet, a skateboard helmet, a hockey helmet, a scooter helmet, and a military helmet,
the sound device may be any one of a headphone, an earphone, a headset, and a microphone,
the glasses or goggles may be any one of glasses, goggles, and an eyesight enhancer,
the VR device may be a VR device,
the jaw assist device may be any one of a jaw corrector, a lifting band, and an open mouth prevention band, and
the cosmetic facial support may be any one of a mask pack and a mask.

In order to fix the main body as the result of being brought into tight contact with the first fixing region and/or the second fixing region,
1) the first fixing member and/or the second fixing member may press at least a part of the first fixing region of the user and/or at least a part of the second fixing region of the user so as to be fixed, or
2) a separate fixing patch may be attached to the at least a part of the first fixing region and/or the at least a part of the second fixing region, and the fixing patch and the at least a part of the first fixing region and/or the at least a part of the second fixing region may be fixed by at least one of a Velcro tape, magnetic force, a hook, adhesion, pressing, a button, a coupling protrusion, a clip, tongs, sliding, binding applied to snowboard shoes, a cable tie, a rail, a button, docking, a bolt and nut, and a string.

The first fixing member and/or the second fixing member may press the first fixing region and/or the second fixing region of the user at a pressure of 40 kPa or less, preferably 0.1 to 10 kPa, more preferably 0.1 to 5 kPa, yet more preferably 0.1 to 1 kPa.

The first fixing member and/or the second fixing member may include a material capable of transmitting electrical stimulation to a human body or a material capable of generating electrical stimulation.

The main body may have a weight of 1 kg or less, preferably 450 g or less.

The present invention provides a method of fixing the apparatus to the head of the user.

The present invention may be provided in the form of reduplication and combination of the above solving means.

### [ Description of Drawings]

FIG. 1 is a schematic view showing positions at which pain depending on wearing of a mask was observed.
FIG. 2 is a side view of a skull including a styloid process.
FIG. 3 is an anatomical chart simultaneously showing a skeleton and a muscle in order to simultaneously show a temporal process and a medial pterygoid.
FIG. 4 is an anatomical chart simultaneously showing a skeleton and a muscle in order to simultaneously show muscles of mastication and a medial pterygoid.
FIG. 5 is an anatomical chart simultaneously showing a skeleton and a muscle in order to simultaneously show a zygomaticus major and a medial pterygoid.
FIG. 6 is a view including a square and small squares formed by dividing the interior of the square provided based on a face of a user having an angle of the mandible and a zygomatic process as one surface.
FIG. 7 is a schematic view showing values of (x,y) based on the face of the user according to the present invention.
FIG. 8 is an illustrative photograph showing that the square provided based on the face of the user including the small squares on the face of the user is actually applied to the face of a person in order to conduct an experiment.
FIG. 9 is a schematic view of an apparatus configured to measure a PPT.
FIG. 10 is a view showing experimental results on a PPT for first to fifth fixing regions according to a first example of the present invention.
FIG. 11 is a view showing experimental results on a VAS for first to fifth fixing regions according to a first example of the present invention.
FIG. 12 is a view showing analysis results on a PPT/VAS for first to fifth fixing regions according to a first example of the present invention.
FIG. 13 is a view showing experimental results on a PPT for first to sixth fixing regions according to a second example of the present invention.
FIG. 14 is a view showing an apparatus configured to measure the degree of fixing when a frame is fixed to a specific region of an actual user at a specific pressure.
FIG. 15 is a view showing a frame having a second fixing member added thereto.
FIG. 16 is a photograph showing that the second fixing member is located above alar creases, on the alar creases, and under the alar creases.
FIGS. 17 to 22 are views showing various examples of a fixing region and a fixing member configured to press the fixing region in an apparatus fixed to a face according to the present invention.

### [ Best Mode]

Now, preferred embodiments of the present invention will be described in detail with reference to the accompanying drawings such that the preferred embodiments of the present invention can be easily implemented by a person having ordinary skill in the art to which the present invention pertains. In describing the principle of operation of the preferred embodiments of the present invention in detail, however, a detailed description of known functions and configurations incorporated herein will be omitted when the same may obscure the subject matter of the present invention.

In addition, the same reference numbers will be used throughout the drawings to refer to parts that perform similar functions or operations. In the case in which one part is said to be connected to another part in the entire specification, not only may the one part be directly connected to the other part, but also, the one part may be indirectly connected to the other part via a further part. In addition, that a certain element is included does not mean that other elements are excluded, but means that such elements may be further included unless mentioned otherwise.

Hereinafter, the present invention will be described with reference to embodiments. However, the embodiments are provided merely to illustratively describe the present invention, and therefore the scope of the present invention is not limited by the embodiments.

The present invention provides an apparatus fixed to a face. Specifically, a main body of the apparatus fixed to the face provided by the present invention includes at least one of a mask, a hat, a helmet, a portable sound device, glasses or goggles, an eye bandage, a VR device, a jaw assist device, and a cosmetic facial support.

In the present invention, the mask covers a lower end of a front of the face, the hat covers at least a part of an upper end of a head, the helmet protects at least a part of the upper end of the head, the portable sound device is a device that transmits sound to ears or the vicinity of the ears using sound waves or a microphone, the glasses or the goggles are provided to correct eyesight or protect eyes, the VR device is a device that enables a user to experience virtual reality using sight and hearing, the jaw assist device is a device that continuously applies pressure to a part of the jaws for medical or cosmetic purposes, and the cosmetic facial support is provided to hide or decorate the face.

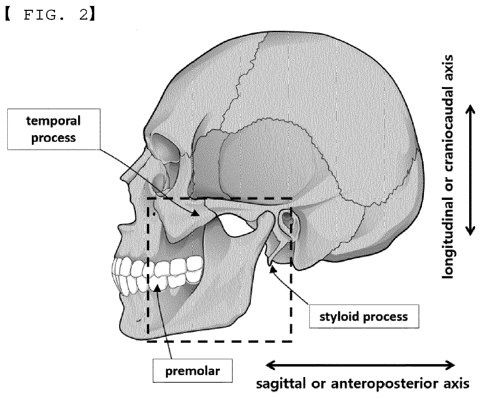

FIGS. 2 to 7 are anatomical charts related to a human body provided to describe fixing regions according to the present invention.

The present invention provides an apparatus fixed to a face using a common improvement method in which various kinds of devices fixed to the face can be fixed to the face of the human body.

Hereinafter, fixing regions will be described with reference to FIGS. 2 to 7.

A fixing region according to FIG. 2 is a lower facial region including a range from a position including a styloid process of a user to a premolar of the user in consideration of a movement range of a sagittal or anteroposterior axis and including a temporal process of the user in consideration of a movement range of a longitudinal or craniocaudal axis.

Here, the sagittal or anteroposterior axis corresponds to an x axis or a lateral axis in FIGS. 2 to 7, and the longitudinal or craniocaudal axis corresponds to a y axis in FIGS. 2 to 7. Specifically, a dotted region illustrated in FIG. 2 may be a 1-1 fixing region according to the present invention.

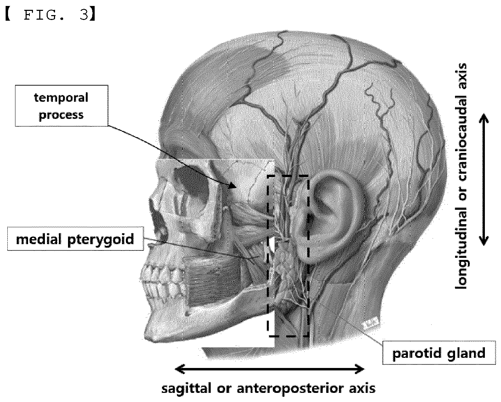

A 1-2 fixing region according to FIG. 3 is a lower facial region including a range from a position including a parotid gland of the user to a region left by excluding a region in which a funiculus anterior is located from two branches of a medial pterygoid of muscles of mastication of the user in consideration of the movement range of the sagittal or anteroposterior axis and including the temporal process of the user in consideration of the movement range of the longitudinal or craniocaudal axis. When compared to FIG. 2, the same movement range of the longitudinal or craniocaudal axis is provided, and there is a difference in that the fixing region includes the range from the position including the parotid gland of the user to the region left by excluding the region in which the funiculus anterior is located from the two branches of the medial pterygoid of the muscles of mastication of the user in consideration of the movement range of the sagittal or anteroposterior axis.

In FIG. 3, two medial pterygoids are finally designated, and a portion corresponding to the front of the face is a region in which the funiculus anterior is located, which is excluded. A dotted region illustrated in FIG. 3 may be the 1-2 fixing region according to the present invention.

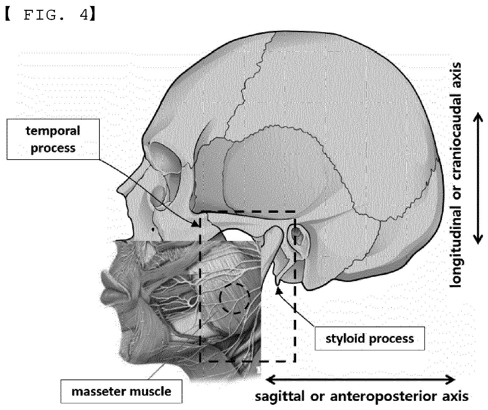

A 1-3 fixing region according to FIG. 4 is a lower facial region including a range from the position including the styloid process of the user to a masseter muscle of the muscles of mastication of the user in consideration of the movement range of the sagittal or anteroposterior axis and including the temporal process of the user in consideration of the movement range of the longitudinal or craniocaudal axis, wherein a muscle belly, which is the most protruding portion of the masseter muscle, is excluded. An apparatus fixed to a face having the above characteristics is provided.

In FIG. 4, the masseter muscle of the muscles of mastication is included; however, the user may easily feel pain in the muscle belly, which is the most protruding portion of the masseter muscle, when pressure is applied thereto, and therefore it is preferable to exclude the muscle belly.

A quadrangular dotted region illustrated in FIG. 4 excluding a circular dotted region is a preferable region of the 1-3 fixing region according to the present invention.

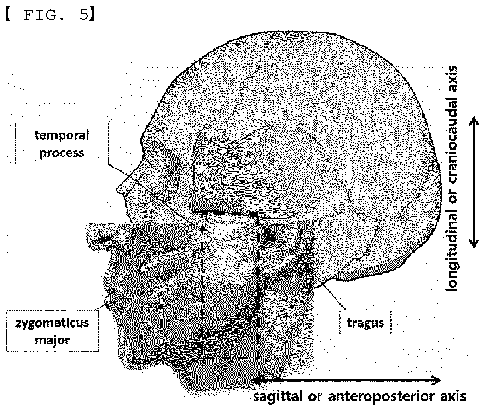

A 1-4 fixing region according to FIG. 5 is a lower facial region including a range from a front end of a tragus of the user to a region in which a rear surface of a zygomaticus major of the user is not pressed in consideration of the movement range of the sagittal or anteroposterior axis and including the temporal process of the user in consideration of the movement range of the longitudinal or craniocaudal axis.

A dotted region illustrated in FIG. 5 may be the 1-4 fixing region according to the present invention.

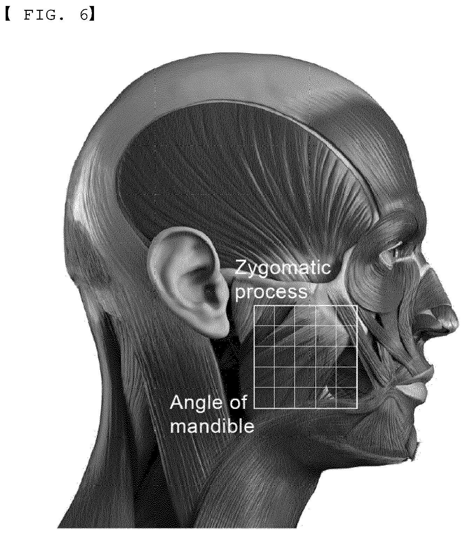

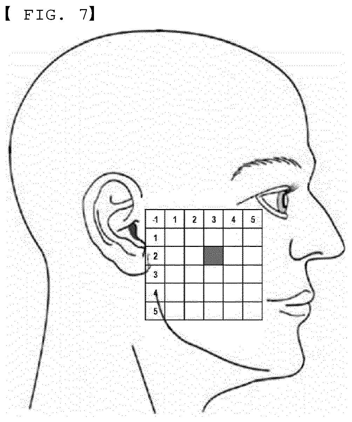

FIG. 6 shows a 1-5 fixing region according to the present invention, and FIG. 7 shows a 1-6 fixing region according to the present invention.

FIG. 6 is a view including a square and small squares formed by dividing the interior of the square provided based on the face of the user having an angle of the mandible and a zygomatic process as one surface, wherein the largest square including 25 small squares may be the 1-5 fixing region according to the present invention.

FIG. 7 shows a 1-6 fixing region according to the present invention, which is an extension of the 1-5 fixing region, wherein values of (x,y) based on the face of the user applied to the 1-5 fixing region and the -6 fixing region are shown.

Specifically, in the 1-5 fixing region, the values of (x,y) based on the face of the user are (1,1), (2,1), (3,1), (4,1), (1,2), (2,2), (3,2), (1,3), (1,4), (1,5), and (2,5), preferably (2,2), (4,2), (1,3), (3,3), (4,3), (5,3), (1,4), (2,4), (3,4), (4,4), (5,4), (1,5), (2,5), (4,5), and (5,5), more preferably (2,1), (2,2), (1,3), (4,3), (5,3), (1,4), (2,4), (4,4), (5,4), (1,5), (2,5), and (5,5), yet more preferably (2,2), (1,3), (2,4), (1,5), and (2,5), most preferably (2,2) and (1,3).

In the 1-6 fixing region, the values of (x,y) based on the face of the user are (-1,-1), (1,-1), (2,-1), (3,-1), (4,-1), (-1,1), (1,1), (2,1), (-1, 2), (1,2), (-1,3), and (1,3).

Here, the square provided based on the face of the user is a square having an angle of the mandible and a zygomatic process of the user as one surface, wherein the square provided based on the face of the user has the sagittal or anteroposterior axis and the longitudinal or craniocaudal axis as a width and a length, respectively, and the distance from the angle of the mandible to the zygomatic process along the longitudinal or craniocaudal axis is the length of one side.

Here, (x,y) provided based on the face of the user is the position of each small square formed when the width and the length of the square provided based on the face of the user are divided into five equal parts, wherein x is designated as 1, 2, 3, 4, and 5 from a rear surface to a front surface of the head of the user, and y is designated as 1, 2, 3, 4, and 5 from an upper surface to a lower surface of the head of the user.

x of the small square of the square provided based on the face of the user further disposed in symmetry based on the longitudinal or craniocaudal axis of the rear surface of the head of the user is designated as a negative value, y of the small square of the square provided based on the face of the user further disposed in symmetry based on the lateral axis of the upper surface of the head of the user is designated as a negative value, and x and y of the small square of the square provided based on the face of the user further disposed in symmetry based on apexes of the upper surface and the rear surface of the head of the user are designated as negative values.

FIG. 6 shows a square provided based on the face of the user having the angle of the mandible and the zygomatic process as one surface, wherein the square provided based on the face of the user has the sagittal or anteroposterior axis and the longitudinal or craniocaudal axis as a width and a length, respectively, and the distance from the angle of the mandible to the zygomatic process along the longitudinal or craniocaudal axis is the length of one side. The square provided based on the face of the user shown in FIG. 6 includes small squares obtained by dividing the width and the length of the square into five equal parts, respectively.

FIG. 7 is a schematic view illustrating values of (x,y) based on the face of the user according to the present invention. In FIG. 7, the square parts having no numbers are identical to the square provided based on the face of the user shown in FIG. 6. In FIG. 7, the numbers shown at the upper end along the lateral axis indicate x values, among the values of (x,y) based on the face of the user, and the numbers shown along the longitudinal or craniocaudal axis indicate y values, among the values of (x,y) based on the face of the user. A portion denoted by -1 has an x value of -1 and a y value of -1, and (x,y) based on the face of the user is (-1,-1). The values of (x,y) of the portions denoted by -1, 1, 2, 3, 4, and 5 along the lateral axis based on the face of the user correspond to (-1,-1), (1,-1), (2,-1), (3,-1), (4,-1), and (5,-1), and the values of (x,y) of the portions denoted by -1, 1, 2, 3, 4, and 5 along the longitudinal or craniocaudal axis based on the face of the user correspond to (-1,-1), (-1,1), (-1,2), (-1,3), (-1,4), and (-1,5). The other portions denoted by no numbers are combinations of x values and y values. For example, the value of (x,y) of the colored portion based on the face of the user is (3,2).

The following experiments were conducted in order to present definite criteria for user discomfort and fixing force depending on specific positions of the fixing regions.

### <First example: Measurement of fixing force in 1-5 fixing region>

The pressure at which the user feels pain (PPT) and the pain that the user feels under fixed pressure (VAS) were measured for the coordinates of values of (x,y) based on the face of the user shown in FIG. 6.

The reason that the pressure at which the user feels pain and the pain that the user feels under fixed pressure are separately measured is that, when pressure is increased, fixing force may be secured but the user may feel pain, or the user may feel no pain but fixing force may not be secured, depending on whether a muscle exists based on the position of the face of the user, the position of a skeleton, and the thickness of a skin.

The pressure pain threshold (PPT) of the user was measured for the coordinates of values of (x,y) based on the face of the user shown in FIG. 6. In this experiment, the pressure pain threshold (PPT) was defined as the maximum pressure value at which the user could endure pain.

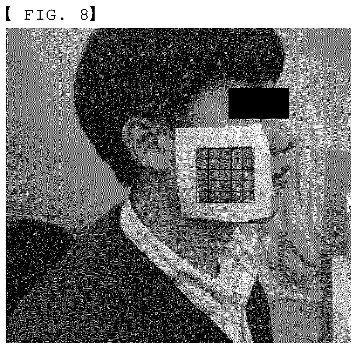

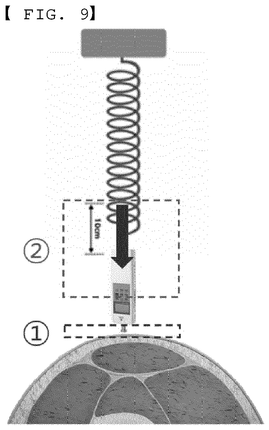

FIG. 8 is an illustrative photograph showing that the square including the small squares on the face of the user is actually applied to the face of a person in order to conduct the experiment, and FIG. 9 is a schematic view of an apparatus configured to measure the PPT.

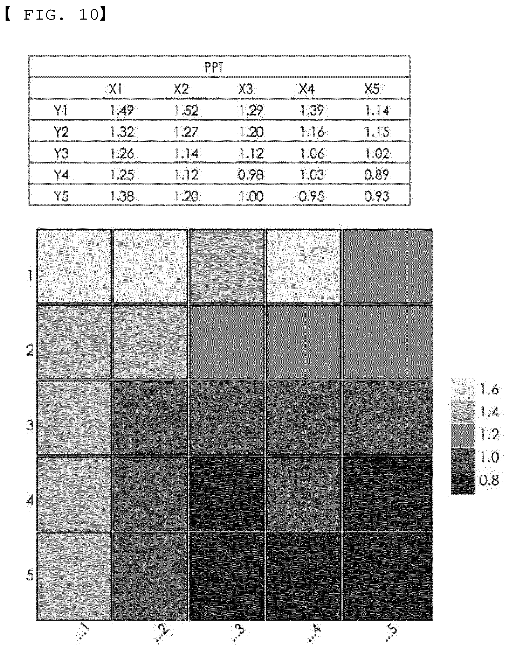

In order to measure distribution in pressure at which the user felt the pain, the pressure at which the user felt the pain (pressure pain threshold: PPT) was measured using an algometer. The pressure pain threshold (PPT) was measured on a small square area corresponding to each of the values of (x,y) based on the face of the user according to the present invention located on a surface of a face of a subject using a PPT instrument. The unit of pressure at which the pain is felt is kg/cm². Pressure values at which pain started to be felt were input to the respective coordinates, and the results of conversion of the pressure values into relative colors are shown in FIG. 10. The results shown in FIG. 10 are the averages of the pressure values measured for 19 people. In FIGS. 10, 11, and 12, upper tables show measured values depending on the positions of (x,y) based on the face of the user shown in Fig. 6, and lower graphs show the measured value using different colors. 1 to 5 marked at the square of each lower graph along the longitudinal axis correspond to y values of the coordinates of values of (x,y) based on the face of the user, and 1 to 5 marked at the square of each lower graph along the lateral axis correspond to x values of the coordinates of values of (x,y) based on the face of the user.

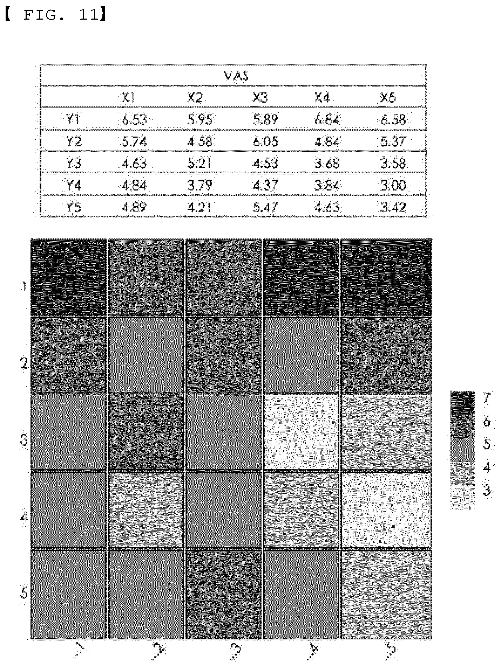

In order to measure distribution in pain that was felt under fixed pressure, discomfort scores (VAS) of the subject were measured using an algometer connected to the coordinates of the respective small squares via a spring in the state in which uniform tension was maintained. The pressure was fixed at 1 kg/cm², discomfort scores were input to the respective coordinates, and the results of conversion of the input values into relative colors are shown in FIG. 11. A visual analog scale (VAS) is a method of setting the pain that patients perceive to levels ranging from 0 indicating "no pain" to 10 indicating "extreme pain" and directly displaying the level of pain for each patient, which is one of pain evaluation scales. The results of FIG. 11 are the averages of the values measured for 19 people.

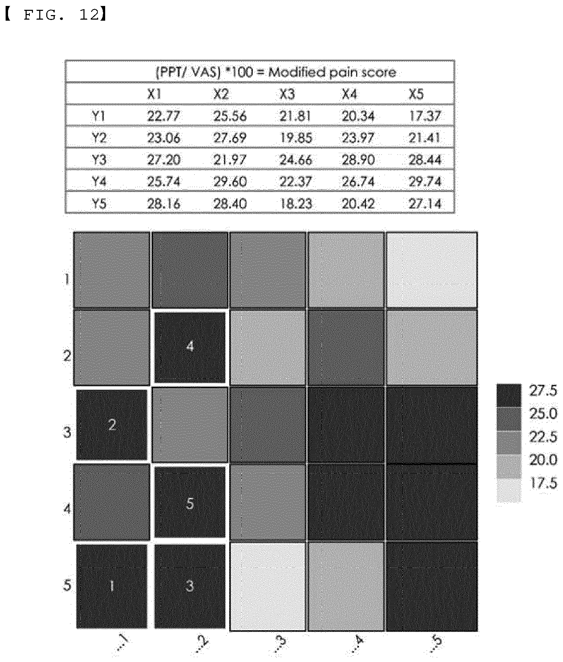

Meanwhile, a novel pain index was derived based on the experimental results on the pressure at which the user felt pain and the pain that the user felt under fixed pressure. The novel pain index, which is a value obtained by multiplying a value obtained by dividing the pressure at which the user feels pain (PPT) by the value of pain that the user feels under fixed pressure (VAS) by 100, is used to select a region in which the user can feel less pain while enduring high pressure. The results of analysis on the coordinates of the respective small squares are shown in FIG. 12.

In the first example of the present invention for the 1-5 fixing region, definite fixing regions could be derived using the three methods. It was evaluated that fixing force could be secured while user discomfort was minimized when a value of 1.2 or more was applied in FIG. 10, when a value of 5.0 or more was applied in FIG. 11, and when a value of 25 or more was applied in FIG. 12.

### <Second example: Measurement of fixing force in 1-6 fixing region>

As described above, the 1-5 fixing region was selected through the first example. The 1-5 fixing region corresponds to the interior of the square provided based on the face of the user having the angle of the mandible and the zygomatic process of the user as one surface.

During research according to the present invention, it was found that a space from the front of an ear canal to the rear of a temporomandibular joint could be utilized as a fixing region. In the second example of the present invention, further evaluation on this region was performed. In extending a range of the fixing region to the rear of the face, it was recognized that utilization was possible unless the range of the fixing region exceeded the temporomandibular joint, the range of the fixing region was extended. In the same manner, the range of the fixing region was extended to the upper part of the face.

The pressure at which the user felt pain (PPT) was measured for the coordinates of values of (x,y) based on the face of the user shown in FIG. 7. A measurement method was identical to the measurement method in the first example.

The reason that the pressure pain threshold (PPT) of the user is measured according to the coordinates of (x,y) based on the face of the user according to the present invention is that the degree of pain may be changed depending on whether a muscle exists based on the position of the face of the user, the position of the skeleton, and the thickness of the skin. That the pressure pain threshold (PPT) is high means that it is possible to increase pressure at which fixing force can be secured.

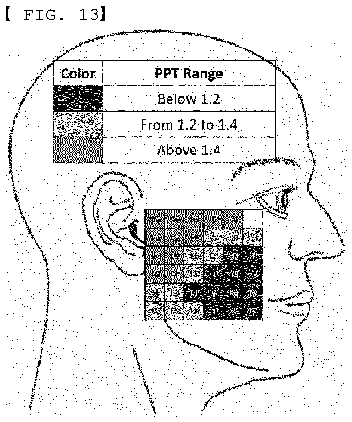

The unit of pressure at which the pain is felt is kg/cm². The pressure pain threshold was input to each of the coordinates, and the results thereof are shown in FIG. 13. The results shown in FIG. 13 are the averages of the values measured for nine people. The upper part of FIG. 13 shows criteria based on which colors are changed depending on a range of pressure pain thresholds (PPT). Below 1.2 means an area that has too low a pressure pain threshold and that is not suitable to be used as a fixing region, and above 1.4 means an area that has the highest pressure pain threshold and that is most suitable to be used as a fixing region.

### <Fixing force experiment>

Among the regions derived according to the first example of the present invention, particularly five regions evaluated to have a high ratio of fixing force to pain were selected, and the fixing force thereof was measured using various methods.

(1,5), (1,3), (2,5), (2,2), and (2,4), which were the values of (x,y) based on the face of the user, were designated as region 1, region 2, region 3, region 4, and region 5, respectively, and fixing force experiments were separately performed thereon. For region 1, region 2, region 3, region 4, and region 5, a frame-shaped semicircular frame (hereinafter referred to as a "frame") that pressed only the coordinates of the small squares corresponding thereto while wrapping the face was manufactured, whether the semicircular frame was maintained in position when "a-e-i-o-u" were pronounced while the pressure at which the semicircular frame pressed the coordinates was changed (Verbal), whether the semicircular frame was maintained in a horizontal state when the semicircular frame was held horizontally, a neck of the user was bent by 30 degrees, and the neck of the user was returned to the original state (Flexion), and whether the semicircular frame got out of position when the user walked at a speed of 3 km/h and 5 km/h (Walking) were observed. The experimental results thereof are shown in Table 1, Table 2, and Table 3. These are the averages of the results of experiments performed for three people.

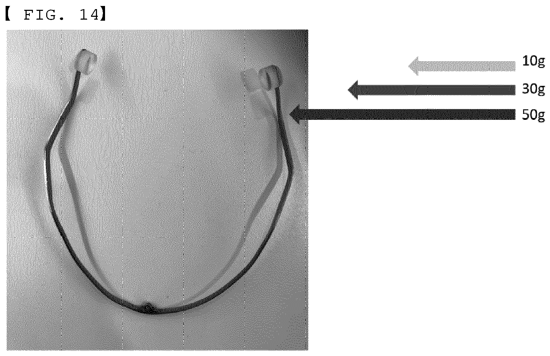

FIG. 14 is a view showing an apparatus configured to measure the degree of fixing when a frame is fixed to a specific region of an actual user at a specific pressure. Additionally, different levels of pressure applied to the face of the user when the elastic force of the apparatus was changed to 10 g, 30 g, and 50 g are schematized.

The pressure described in Table 1, which is elastic force or force that the frame applies to the user, and the unit of the pressure is g. Table 1 shows the minimum pressure necessary for the frame to be maintained in position during the verbal, flexion, and walking experiments, and it can be seen that, at position 2 and position 4, low levels of pressure were applied to the frame and the frame was maintained in position.

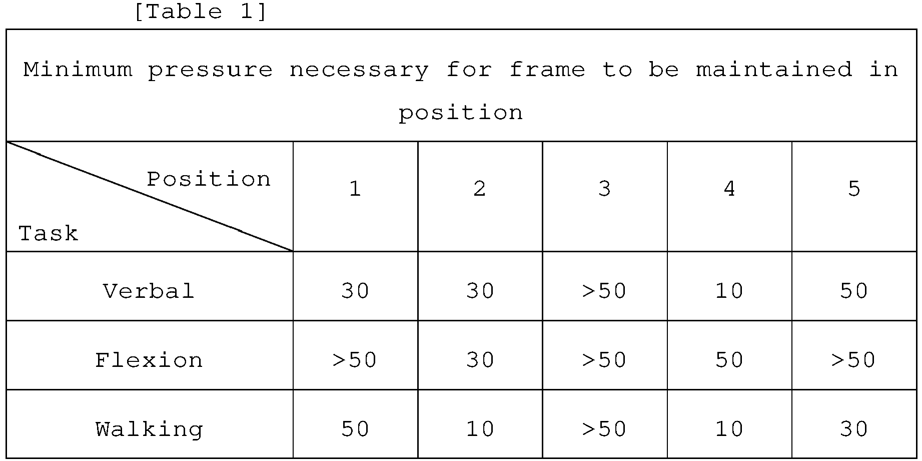

Table 2 shows the number of people having frames deviated from the original positions according to the levels of fixing pressure during the verbal, flexion, and walking experiments. Similarly to Table 1, the frames did not deviate from the original positions at position 2 and position 4.

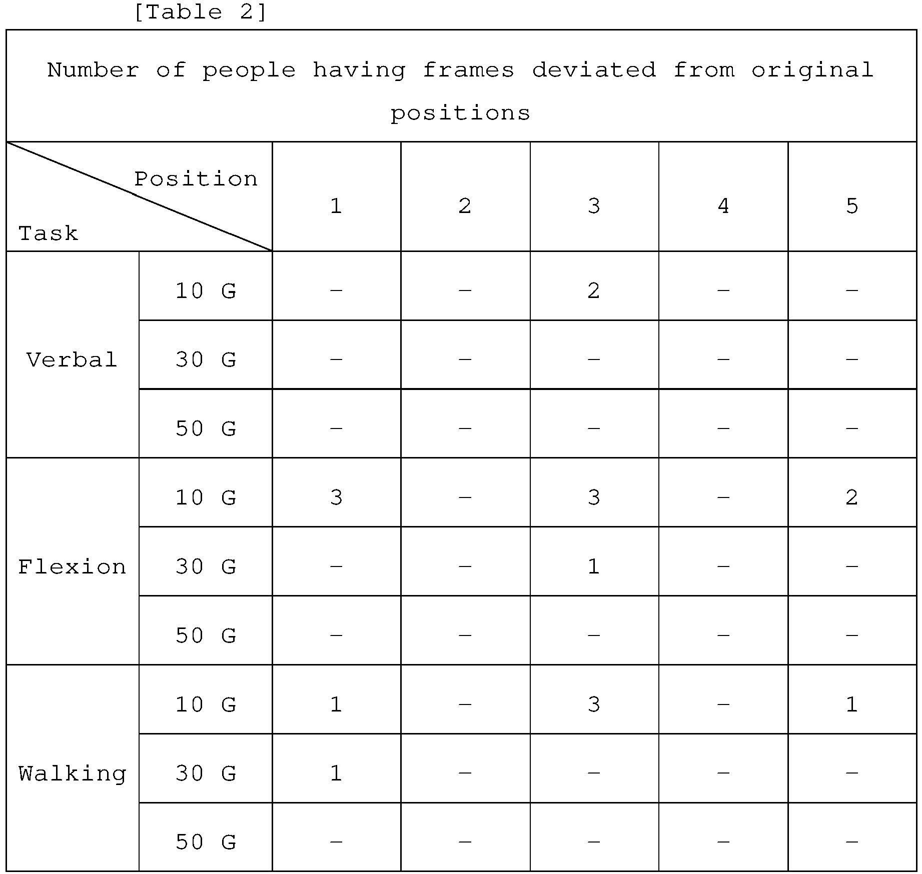

Table 3 shows the region in which the frames did not move depending on the levels of pressure applied by the frames, and it can be seen that, at position 2 and position 4, the frame did not move even at low levels of pressure.

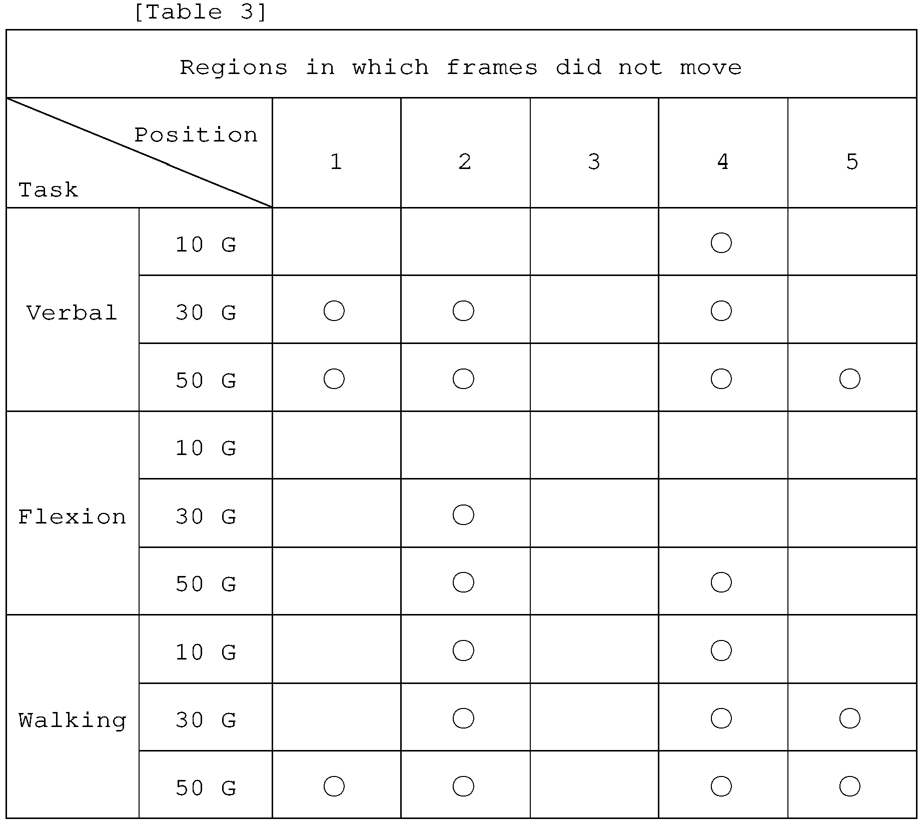

For the five positions, weights having predetermined weights were suspended from the frame in order to measure whether the frame was fixed, and the results are shown in Table 4.

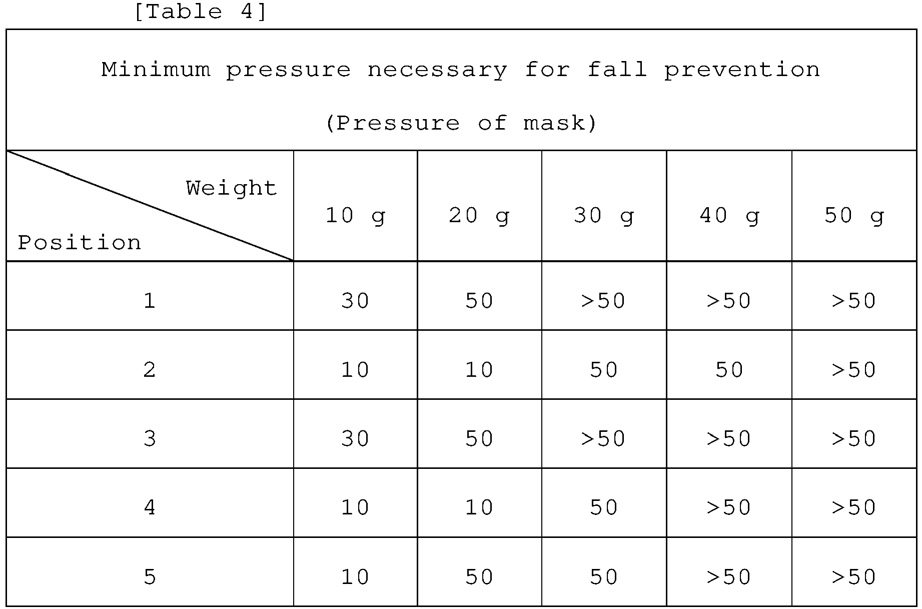

As can be seen from Table 4, the heavy weights can be fixed even at low pressure at position 2 and position 4. It can be seen from the above experiment that, at position 2 and position 4, the highest fixing force is secured.

### <Measurement of fixing force in second fixing region>

A fixing force effect due to addition of a second fixing region was measured.

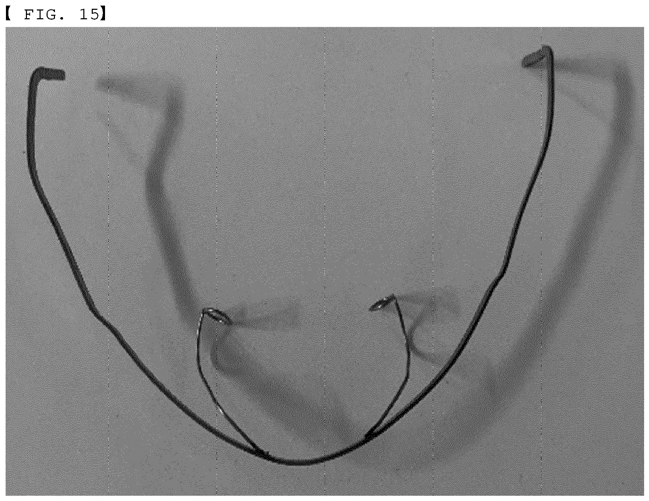

FIG. 15 is a view showing a second fixing member added to the frame in order to measure fixing force due to addition of a second fixing region. After the frame was fixed at position 2 and position 4, fixing force was observed while the second fixing member was added or not and while the second fixing member was located above the alar creases, on the alar creases, and under the alar creases.

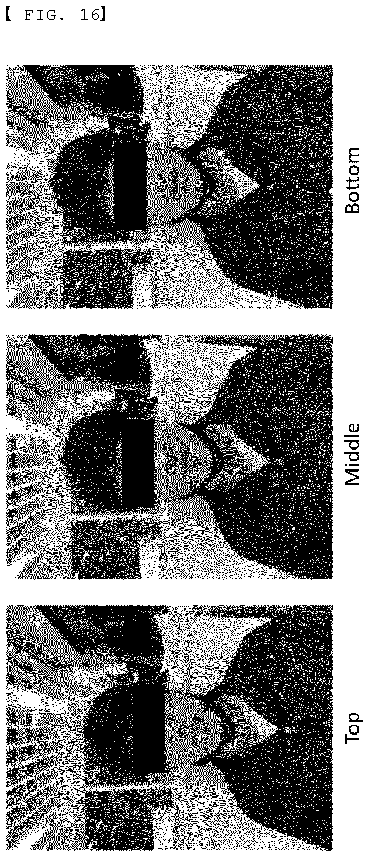

FIG. 16 is a photograph showing that the second fixing member is located above the alar creases, on the alar creases, and under the alar creases.

In the same manner as the fixing force was measured with only the frame, whether the semicircular frame got out of position when "a-e-i-o-u" were pronounced (Verbal) and whether the semicircular frame got out of position when the user walked at a speed of 3 km/h and 5 km/h (Walking) were observed in the state in which separate weights of 10, 20, 30, 40, and 50 g were added to the frame. The results are the averages of the results of experiments performed for three people.

As can be seen with reference to Table 2, when the fixing force of the frame was 30 g, the frame did not deviate from the fixing position for Verbal and Walking irrespective of whether the second fixing member was added, at position 2 and position 4.

In order to observe the effect based on the second fixing member, the support pressure of the frame was minimized and the pressure of the frame was reduced to 10 g or less. In this case, 10 g, which is the minimum weight, cannot be supported even at position 2 and position 4. Table 5 below shows the experimental results according thereto. The cases in which the frame did not deviate from the original position depending on the weight of the weights and the position of the second fixing member are denoted by ○.

**[Table 5]**

| | With | | | | | | Without | |
|---|---|---|---|---|---|---|---|---|
| | Above | | On | | Under | | - | |
| Weight/Position | 2 | 4 | 2 | 4 | 2 | 4 | 2 | 4 |
| 10 g | | ○ | ○ | ○ | ○ | ○ | | |
| 20 g | | | ○ | ○ | ○ | ○ | | |
| 30 g | | | | | ○ | ○ | | |
| 40 g | | | | | ○ | ○ | | |
| 50 g | | | | | ○ | ○ | | |

Referring to Table 5, for the frame having no second fixing member, the frame got out of position at position 2 and position 4. In addition, the extent to which the second fixing member deviated from the original position was changed depending on the position thereof, and it was observed that, when the second fixing member was located under the alar creases, the strongest fixing force was secured.

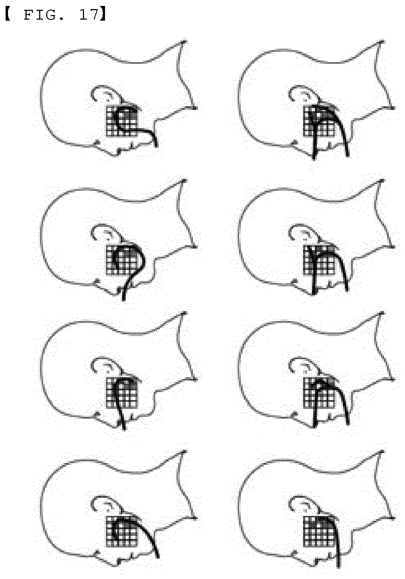

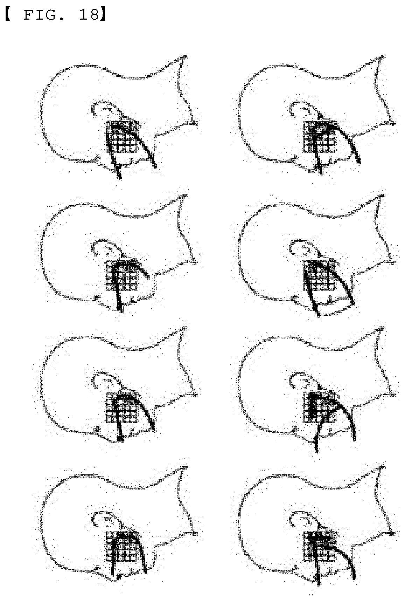

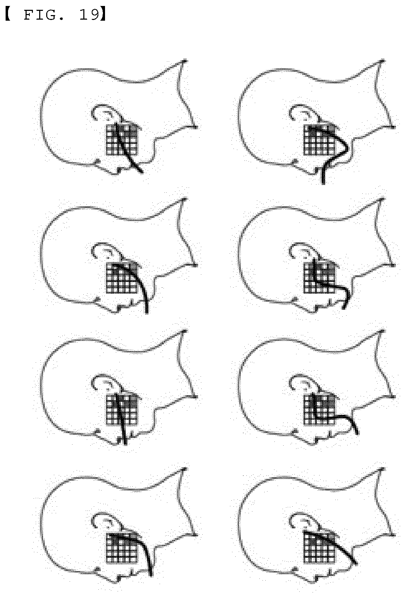

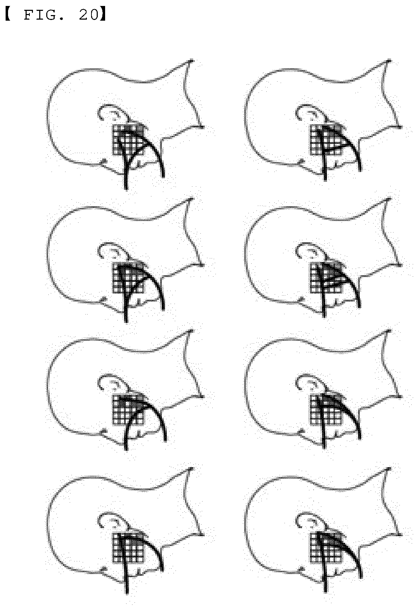

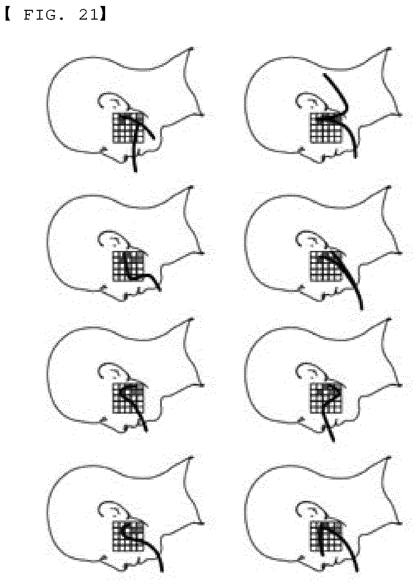

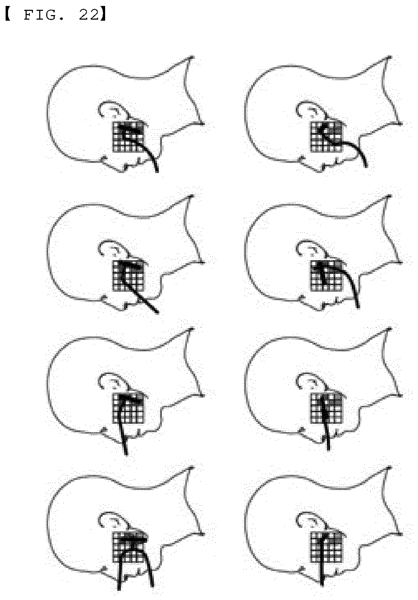

FIGS. 17 to 22 are views showing various examples of the fixing region and the fixing member configured to press the fixing region in the apparatus fixed to the face according to the present invention.

In FIGS. 17 to 22, gray regions indicate regions in which the fixing member can be brought into tight contact with the face of the user while directly facing the face of the user, a black solid line indicates an illustration of the fixing member, and various embodiments of a frame made of an elastic material are shown. In addition to the gray regions, the frame may be bent so as to directly press the face. In the figures, the frame is shown as only a line; however, change to an elastic surface is possible, and therefore a concrete illustration thereof will be omitted. Furthermore, the portion of the frame that is bent so as to directly press the face may also be changed into a surface.

FIGS. 17 to 22 show only the section of the face; however, the frame is configured to wrap the face, and therefore the fixing member is also disposed at the portion that is not shown in the figures.

### [ Industrial Applicability]

The present invention may provide an apparatus fixed to a face as the result of being brought into tight contact with a specific region of the face. Compared to a conventional apparatus, including a mask, the apparatus fixed to the face is characterized in that 1) detachment and attachment of the apparatus are easy, 2) contact between the apparatus and the skin, including the face, during detachment and attachment of the apparatus is minimized, 3) skin irritation is low even though the apparatus is worn for a long time, 4) discomfort due to pain in other musculoskeletal systems, including ears, is minimized, 5) a pressed region is clearly specified and the region is optimized, whereby consistent fixing force is provided regardless of user, and 6) physical force for the ears is not applied to the specific region of the face that is pressed in the present invention, as previously described, whereby there are no problems of stress due to long-term wearing and concentration weakening.

## Claims

1. An apparatus fixed to a face, the apparatus comprising:
a main body located at a front surface or a rear surface of a head of a user; and
a first fixing member connected to the main body, the first fixing member being brought into tight contact with at least a part of a first fixing region of the user in order to fix the main body, wherein
in the face of the user, the first fixing region comprises at least one of:
1) a 1-1 fixing region as a lower facial region comprising a range from a position comprising a styloid process of the user to a premolar of the user in consideration of a movement range of a sagittal or anteroposterior axis and
comprising a temporal process of the user in consideration of a movement range of a longitudinal or craniocaudal axis;
2) a 1-2 fixing region as a lower facial region comprising a range from a position comprising a parotid gland of the user to a region left by excluding a region in which a funiculus anterior is located from two branches of a medial pterygoid of muscles of mastication of the user in consideration of the movement range of the sagittal or anteroposterior axis and
comprising the temporal process of the user in consideration of the movement range of the longitudinal or craniocaudal axis;
3) a 1-3 fixing region as a lower facial region comprising a range from the position comprising the styloid process of the user to a masseter muscle of the muscles of mastication of the user in consideration of the movement range of the sagittal or anteroposterior axis and
comprising the temporal process of the user in consideration of the movement range of the longitudinal or craniocaudal axis, wherein a muscle belly, which is a most protruding portion of the masseter muscle, is excluded;
4) a 1-4 fixing region as a lower facial region comprising a range from a front end of a tragus of the user to a region in which a rear surface of a zygomaticus major of the user is not pressed in consideration of the movement range of the sagittal or anteroposterior axis and
comprising the temporal process of the user in consideration of the movement range of the longitudinal or craniocaudal axis;
5) a 1-5 fixing region as an interior of a square provided based on the face of the user, wherein
the square provided based on the face of the user is a square having an angle of mandible and a zygomatic process of the user as one surface, and wherein the square provided based on the face of the user has the sagittal or anteroposterior axis and the longitudinal or craniocaudal axis as a width and a length, respectively, and a distance from the angle of the mandible to the zygomatic process along the longitudinal or craniocaudal axis is a length of one side; and
6) a 1-6 fixing region configured such that values of (x,y) based on the face of the user are (-1,-1), (1,-1), (2,-1), (3,-1), (4,-1), (-1,1), (1,1), (2,1), (3,1), (4,1), (5,1), (-1, 2), (1,2), (2,2), (3,2), (-1,3), (1,3), (2,3), (-1,4), (1,4), (-1,5), (1,5), and (2,5), wherein
(x,y) provided based on the face of the user is selected based on coordinates of each small square formed when a width and a length of the square provided based on the face of the user are divided into five equal parts, x is designated as 1, 2, 3, 4, and 5 from the rear surface to the front surface of the head of the user and y is designated as 1, 2, 3, 4, and 5 from an upper surface to a lower surface of the head of the user as a result of division into five equal parts,
x of the small square of the square provided based on the face of the user further disposed in symmetry based on the longitudinal or craniocaudal axis of the rear surface of the head of the user is designated as a negative value,
y of the small square of the square provided based on the face of the user further disposed in symmetry based on a lateral axis of the upper surface of the head of the user is designated as a negative value, and
x and y of the small square of the square provided based on the face of the user further disposed in symmetry based on apexes of the upper surface and the rear surface of the head of the user are designated as negative values.

2. The apparatus according to claim 1, wherein
in the 1-5 fixing region, the values of (x,y) based on the face of the user are (1,1), (2,1), (3,1), (4,1), (1,2), (2,2), (3,2), (1,3), (1,4), (1,5), and (2,5), and
in the 1-6 fixing region, the values of (x,y) based on the face of the user are (-1,-1), (1,-1), (2,-1), (3,-1), (4,-1), (-1,1), (1,1), (2,1), (-1, 2), (1,2), (-1,3), and (1,3).

3. The apparatus according to claim 1, wherein, in the 1-5 fixing region, the values of (x,y) based on the face of the user are (2,2), (4,2), (1,3), (3,3), (4,3), (5,3), (1,4), (2,4), (3,4), (4,4), (5,4), (1,5), (2,5), (4,5), and (5,5).

4. The apparatus according to claim 1, wherein, in the 1-5 fixing region, the values of (x,y) based on the face of the user are (2,1), (2,2), (1,3), (4,3), (5,3), (1,4), (2,4), (4,4), (5,4), (1,5), (2,5), and (5,5).

5. The apparatus according to claim 4, wherein, in the 1-5 fixing region, the values of (x,y) based on the face of the user are (2,2), (1,3), (2,4), (1,5), and (2,5).

6. The apparatus according to claim 4, wherein, in the 1-5 fixing region, the values of (x,y) based on the face of the user are (2,2) and (1,3).

7. The apparatus according to claim 1, further comprising:
a second fixing member connected to the main body, the second fixing member being brought into tight contact with at least a part of a second fixing region of the user in order to fix the main body, wherein
the second fixing region comprises at least one of a nasolabial fold area of the user, a levator labii superioris or a levator labii superioris alaeque nasi of the user, and an outer circumferential part of an alar crease of the user.

8. The apparatus according to claim 7, wherein the second fixing region comprises the outer circumferential part of the alar crease of the user.

9. The apparatus according to claim 1, wherein the styloid process, the premolar, and the temporal process are excluded from the first fixing region.

10. The apparatus according to claim 7, wherein at least two of the main body, the first fixing member, and the second fixing member are made of an identical material.

11. The apparatus according to claim 7, further comprising an additional fixing member brought into tight contact with an additional fixing region of the face of the user other than the first fixing region and/or the second fixing region in order to fix the main body.

12. The apparatus according to claim 7, further comprising:
a first connection portion configured to connect the main body and the first fixing member to each other; and
a second connection portion configured to connect the main body and the second fixing member to each other.

13. The apparatus according to claim 12, wherein the first connection portion and/or the second connection portion has an adjustable length.

14. The apparatus according to claim 12, wherein the main body, the first connection portion, the second connection portion, the first fixing member, and the second fixing member are integrally formed using an identical material.

15. The apparatus according to claim 12, wherein the first connection portion and/or the second connection portion is elastic.

16. The apparatus according to claim 12, wherein each of the first connection portion, the second connection portion, the first fixing member, and the second fixing member is made of at least one of a resin, a metal, a semiconductor, ceramic, leather, paper, fiber comprising glass fiber and carbon fiber, glass, rubber, wood, a biomaterial, and carbon fiber.

17. The apparatus according to claim 7, wherein
the first fixing member brought into tight contact with the at least a part of the first fixing region has at least one first tight contact surface, and
the second fixing member brought into tight contact with the at least a part of the second fixing region has at least one second tight contact surface.

18. The apparatus according to claim 1 or 7, further comprising an auxiliary fixing member fixed to an auxiliary fixing region located at at least one of a nose, jaws, a mastoid bone, a back of a neck, and a parietal bone or an occipital bone of the head in order to fix the main body.

19. The apparatus according to claim 1 or 7, wherein the main body is at least one of a mask, a hat, a helmet, a portable sound device, glasses or goggles, an eye bandage, a VR device, a jaw assist device, and a cosmetic facial support.

20. The apparatus according to claim 19, wherein
the mask is any one of an air pollution mask, a disposable mask, a filter replaceable mask, an industrial mask, a military mask, a gas mask, a surgical mask, a cooking mask, an ultraviolet mask, a cold protection mask, a continuous positive airway pressure mask, an oxygen mask, a welding mask, a hyperventilation prevention mask, a training mask, a scuba diving mask, a skin care mask, an LED mask, an ultrasonic mask, a low frequency mask, and a facial mask,
the hat is any one of a sun cap, a fedora, a baseball cap, and a cap,
the helmet is any one of a bicycle helmet, a ski helmet, a snowboard helmet, a skateboard helmet, a hockey helmet, a scooter helmet, and a military helmet,
the sound device is any one of a headphone, an earphone, a headset, and a microphone,
the glasses or goggles are any one of glasses, goggles, and an eyesight enhancer,
the VR device is a VR device,
the jaw assist device is any one of a jaw corrector, a lifting band, and an open mouth prevention band, and
the cosmetic facial support is any one of a mask pack and a mask.

21. The apparatus according to claim 7, wherein
in order to fix the main body as a result of being brought into tight contact with the first fixing region and/or the second fixing region,
1) the first fixing member and/or the second fixing member presses at least a part of the first fixing region of the user and/or at least a part of the second fixing region of the user so as to be fixed, or
2) a separate fixing patch is attached to the at least a part of the first fixing region and/or the at least a part of the second fixing region, and the fixing patch and the at least a part of the first fixing region and/or the at least a part of the second fixing region are fixed by at least one of a Velcro tape, magnetic force, a hook, adhesion, pressing, a button, a coupling protrusion, a clip, tongs, sliding, binding applied to snowboard shoes, a cable tie, a rail, a button, docking, a bolt and nut, and a string.

22. The apparatus according to claim 21, wherein the first fixing member and/or the second fixing member presses the first fixing region and/or the second fixing region of the user at a pressure of 40 kPa or less.

23. The apparatus according to claim 7, wherein the first fixing member and/or the second fixing member comprises a material capable of transmitting electrical stimulation to a human body or a material capable of generating electrical stimulation.

24. The apparatus according to claim 1 or 7, wherein the main body has a weight of 1 kg or less.

25. A method of fixing the apparatus according to claim 1 or 7 to the head of the user.
